(19) Europäisches Patentamt
European Patent Office
Office européen des brevets

(11) Veröffentlichungsnummer: **0 437 898 A1**

(12) # EUROPÄISCHE PATENTANMELDUNG

(21) Anmeldenummer: **90250323.4**

(51) Int. Cl.[5]: **C07D 207/08**

(22) Anmeldetag: **20.12.90**

(30) Priorität: **21.12.89 DD 336092**

(43) Veröffentlichungstag der Anmeldung: **24.07.91 Patentblatt 91/30**

(84) Benannte Vertragsstaaten: **BE CH DE ES FR GB IT LI NL**

(71) Anmelder: **INSTITUT FÜR CHEMISCHE TECHNOLOGIE**
**Rudower Chaussee 5**
**O-1199 Berlin(DE)**

Anmelder: **ZENTRALINSTITUT FÜR ORGANISCHE CHEMIE**
**Rudower Chaussee 5**
**O-1199 Berlin(DE)**

(72) Erfinder: **Ballschuh, Detlef, Dr.**
**Graudenzer Strasse 17**
**O-1034 Berlin(DE)**
Erfinder: **Ohme, Roland, Dr.**
**Waldstrasse 6**
**O-1180 Berlin(DE)**
Erfinder: **Seibt, Horst, Dr.**
**Eugen-Schönhaar-Strasse 15**
**O-1055 Berlin(DE)**
Erfinder: **Gründemann, Egon, Dr.**
**Waldstrasse 4**
**O-1180 Berlin(DE)**

(54) **3-Chlormethylsulfonylmethyl-4-sulfomethyl-pyrrolidinium-betaine und Verfahren zu ihrer Herstellung.**

(57) Die Erfindung betrifft neue 3-Chlormethylsulfonylmethyl-4-sulfomethyl-pyrrolidinium-betaine (Chlormethylsulfon-sulfobetaine) der allgemeinen Formel I und Verfahren zu ihrer Herstellung. Die Verbindungen nach Formel I knen als organische Zwischenprodukte für weiterführende Synthesen oder, wenn sie einen langkettigen Alkylrest enthalten, als polyfunktionelle Tenside eingesetzt werden. Molare Mengen von Diallylammoniumchlorid werden mit Dichloressigsäure und der doppeltmolaren Menge Natriumhydrogensulfit in Gegenwart einer katalytischen Menge eines Peroxodisulfats miteinander umgesetzt und die erhaltene Reaktionslösung nach Zugabe einer katalytischen Jodidmenge durch Erwärmen zu 3-Chlor-methylsulfonylmethyl-4-sulfomethyl-pyrrolidinium-betainen (Chlormethylsulfon-sulfobetaine) der allgemeinen Formel I umgewandelt.

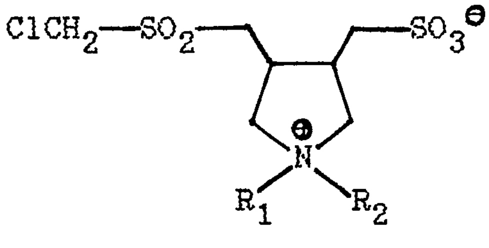

I

EP 0 437 898 A1

## 3-CHLORMETHYLSULFONYLMETHYL-4-SULFOMETHYL-PYRROLIDINIUM-BETAINE UND VERFAHREN ZU IHRER HERSTELLUNG

Die Erfindung betrifft neue 3-Chlormethylsulfonylmethyl-4-sulfomethyl-pyrrolidinium-betaine (Chlormethylsulfon-sulfobetaine) der allgemeinen Formel I,

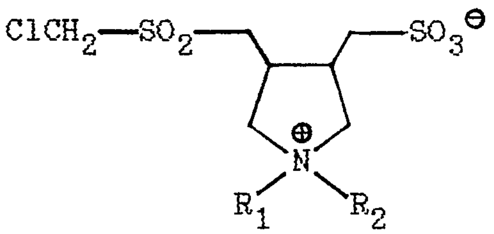

I

worin

$R_1$ und $R_2$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen.

Die neuen Chlormethylsulfon-sulfobetaine der Formel I stellen als organische Zwischenprodukte mit Sulfobetaincharakter einen reaktiven Baustein für weiterführende Synthesen dar. Ist mindestens einer der Substituenten $R_1$ oder $R_2$ ein langkettiger Alkylrest, so können diese Verbindungen als polyfunktionelle Tenside Verwendung finden.

Chlormethylsulfon-sulfobetaine der Formel I sowie Verfahren zu ihrer Herstellung sind bisher nicht bekannt geworden. Nach M.Kulka, J.Amer.chem.Soc.72,1215(1950) sind jedoch Chlormethylarylsulfone bekannt, welche sich bei der Umsetzung von Sulfinsäuren mit Dichloressigsäure unter Sodazusatz und Decarboxylierung bilden. Aus p-Chlorbenzolsulfinsäure wird so z.B das entsprechnde Chlormethylarylsulfon nach 48 stündigem Erwärmen der wäßrigen Lösung der Komponenten in 66 %iger Ausbeute gewonnen.

Seit kurzem stehen neuartige Sulfobetain-sulfinsäuren bzw. deren Salze als potentielle Ausgangsstoffe für die Synthese von Chlormethylsulfon-sulfobetainen der Formel I zur Verfügung.

Derartige Sulfobetainsulfinate werden durch radikalisch initiierte Sulfocyclosulfinierung von Diallylammoniumsalzen mit Hydrogensulfit nach DD 225 128 A1 bzw. EP 163 319 A3 gewonnen.

Versuche, welche nach dem Stand der Technik mit z.B. Natrium-1,1-dimethyl-3-sulfinatomethyl-4-sulfomethyl-pyrrolidinium-betain und Natriumdichloracetat (vgl. Beispiel 1) ausgeführt wurden, ergaben nach langer Reaktionszeit neben nicht völlig umsetzbaren Ausgangsmaterial nur unbefriedigende Ausbeuten an dem Zielprodukt.

Gegenstand der Erfindung ist ein einfaches Syntheseverfahren zur Herstellung von 3-Chlormethylsulfonylmethyl-4-sulfomethyl-pyrrolidinium-betainen aus Sulfobetainsulfinaten bzw. aus deren Vorprodukten, dar es gestattet, die Verfahrensschritte ohne Isolierung von Zwischenprodukten und ohne Bildung von Nebenprodukten bei gleichzeitig hoher Reinheit und Ausbeute des Zielproduktes zu führen.

Erfindungsgemäß werden Diallylammoniumsalze, vorzugsweise Diallylammoniumchloride, der allgemeinen Formel II,

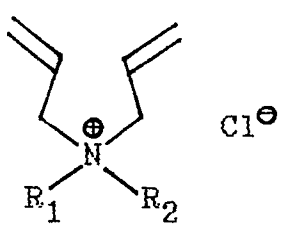

II

in der

$R_1$ und $R_2$ die obengenannten Bedeutungen haben,

mit molaren Mengen Dichloressigsäure und der zweifachmolaren Menge eines Metallhydrogensulfits, vorzugsweise Natriumhydrogensulfit, in Gegenwart einer katalytischen Menge eines Peroxodisulfats in einem ersten Verfahrensschritt miteinander umsetzt und im sich anschließenden zweiten Verfahrensschritt die erhaltene Reaktionslösung nach Zugabe einer katalytischen Menge eines im Reaktionsmedium löslichen Jodids solange zum Sieden erhitzt bis der Sulfinat-Gehalt der reagierenden Lösung auf praktisch Null abgesunken ist bzw. die Decarboxylierung beendet ist.

Die Gesamtreaktion, die radikalisch initiierte Sulfocyclosulfinierung des Diallylammoniumsalzes mit gekoppelter Chlorcarboxymethylierung/Decarboxylierung des in situ gebildeten Sulfobetainsulfinats in zwei Verfahrensstufen, veranschaulicht das folgende Schema:

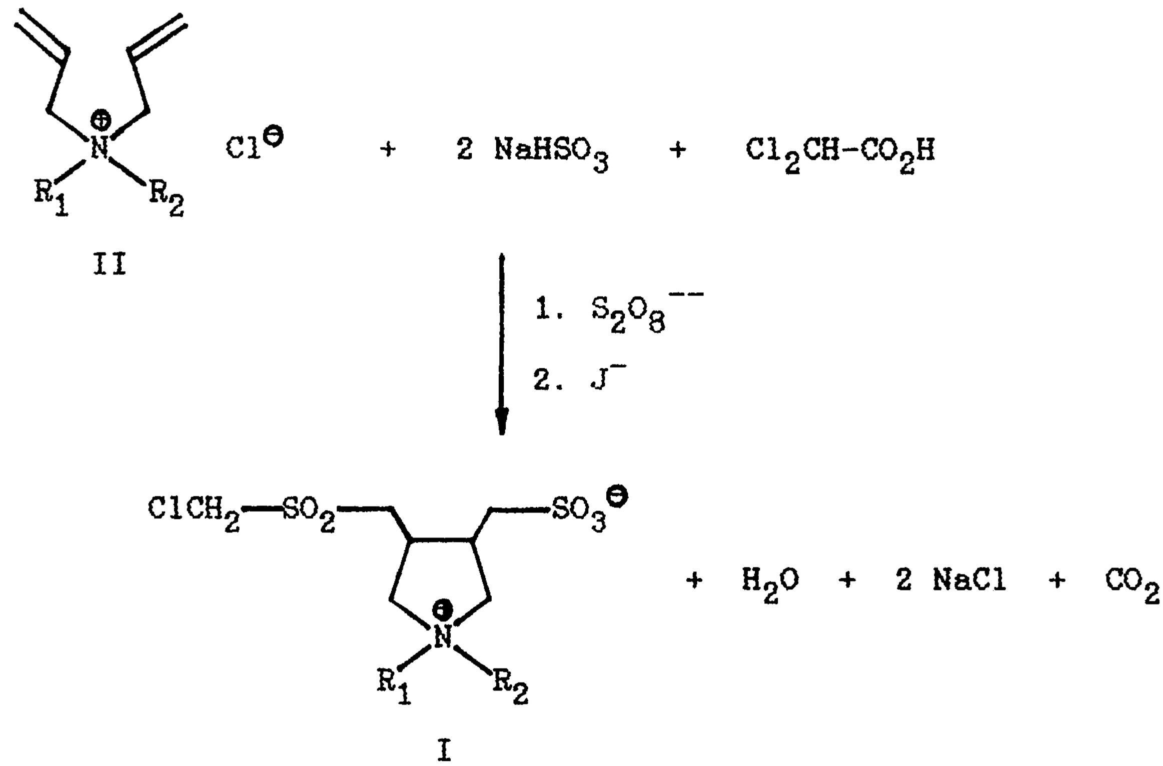


II

I

Die Sulfocyclosulfinierung eines Diallylammoniumsalzes verläuft nach DD 225 128 A1 nur dann mit maximaler Ausbeute an Sulfocyclosulfinierungsprodukt (Sulfobetainsulfinat), wenn pH-Bedingungen um 2 eingehalten werden. Das Einstellen auf diesen PH-Wert wird durch Zugabe einer Mineralsäure zur Mischung aus Diallylammoniumsalz und Hydrogensulfit erreicht. Entsprechende pH-Wert-Einstellungen sind bisher nicht mit Dichloressigsäure vorgenommen worden. Es kann deshalb als ein überraschender Befund angesehen werden daß beispielsweise der Zusatz von 1 mol Dichloressigsäure zu einer Mischung aus 1 mol Diallylammoniumsalz und 2 mol technischer Natriumhydrogensulfitlösung den optimalen Start-pH-Wert um 2 ergibt; also Bedingungen wie sie der obigen Zielstellung entsprechen. Die Reihenfolge des Zusammengebens der Reaktionsteilnehmer ist für den Reaktionsablauf ohne Bedeutung.

In Abhängigkeit von der Qualität der verwendeten Natriumhydrogensulfitlösung können geringe pH-Wert-Schwankungen auftreten. Sie sind dadurch zu korrigieren, daß entweder eine geringe Menge einer Mineralsäure zugesetzt oder die Reaktion mit einer Teilmenge der Halogencarbonsäure gestartet und die Restmenge der Halogencarbonsäure erst während der Reaktion zugesetzt wird.

Bei Einsatz von z.B. tertiären Diallylaminen als Ausgangsprodukte der Umsetzung sollten diese zweckmäßigerweise in der Dichloressigsäure gelöst und nach Zugabe der Hydrogensulfitlösung der Start-pH-Wert mit einer Mineralsäure eingestellt werden. Da bei tertiären Diallylaminen und einem Start-pH-Wert von 2,5 die Sulfocyclosulfinierung bereits vollständig abläuft, ist weniger als die moläquivalente Menge an Mineralsäure für die Bildung des Diallylammoniumsalzes notwendig.

Als Endprodukte der Gesamtreaktion werden dann in Abhängigkeit vom eingesetzten Metallhydrogensulfit die Metallsulfonate entsprechend der Formel I erhalten. Aus diesen kann durch Zusatz von Mineralsäure das Zielprodukt freigesetzt werden.

Zur so vorbereiteten Startlösung, welche eine Temperatur zwischen 20 bis 30 °C haben sollte, wird zur Durchführung des ersten Verfahrensschritts 1 bis 5 Mol-% Peroxodisulfat, bezogen auf das eingesetzte Diallylammoniumsalz, hinzugesetzt, um die Sulfocyclosulfinierungsreaktion auszulösen.

Es hat sich dabei als zweckmäßig erwiesen, das Peroxodisulfat in zwei Portionen hinzuzusetzen, um einen möglichst vollständigen Umsatz zu erreichen, wobei meistens Mengen von 2 mal 2 Mol-%, bezogen auf das eingesetzte Diallylammoniumsalz, völlig ausreichend sind.

Als Peroxodisulfate sind Natrium-, Kalium- oder Ammoniumperoxodisulfat geeignet, welche entweder als Lösung oder pulverförmig eingesetzt werden können.

Die anfänglich fahlgelbe Startlösung färbt sich nach der Peroxodisulfatzugabe rot bis blutrot, hellt sich aber kurz nach Durchlaufen des Temperaturmaximums deutlich auf.

Zur Einleitung des zweiten Verfahrensschrittes wird zur reagierenden Lösung eine katalytische Menge eines löslichen Jodids, z.B. Natrium-, Kalium- oder Ammoniumjodid hinzugefügt und solange unter Siedekühlung weiter erwärmt bis in der reagierenden Lösung durch bromatometrische Titration einer Probe kein Sulfinat mehr nachgewiesen werden kann.

Bei einer Einsatzmenge von 14 bis 20 mmol Natrium-, Kalium- oder Ammoniumjodid/mol Diallylammoniumsalz werden im Verlaufe von ein bis zwei Stunden vollständige Umsätze erreicht. Während Mengen unter 14 mmol der o.g. Jodide keine katalytische Wirkung mehr erkennen lassen, beschleunigen größere Mengen als 20 mmol die Reaktion weiter; verfärben jedoch später die kristallinen Zielprodukte.

Führt man hingegen die Alkylierung des Sulfocyclosulfinierungsprodukts mit Dichloressigsäure ohne Jodidzusatz aus, findet in der Anfangsphase die gewünschte Umsetzung statt. Sie verlangsamt sich aber mit fortschreitender Reaktionszeit immer mehr, so daß auch nach langen Reaktionszeiten keine vollständige Umsetzung erreichbar ist. Gerade aber wegen der unvollständig bleibenden Umsetzung lassen sich aus solchen komplexen Mischungen aufgrund des polaren Charakters von Ausgangs- und Endprodukten die Zielprodukte nur schwer und unvollständig oder gar nicht abtrennen.

Überraschend an diesem zweiten Verfahrensschritt ist ferner die Tatsache , daß sich das Sulfocyclosulfinierungsprodukt des Diallylammoniumsalzes bei niedrigem pH-Wert in der Siedehitze unter Decarboxylierung chlormethylieren läßt, obwohl bekannt ist [W.Jeblick und W.Bunge in "Ullmanns Encyklopädie der technischen Chemie", Bd.22 (1982)), S.315], daß unter solchen Bedingungen - niedriger pH-Wert und hohe Temperaturen - Sulfinsäuren bevorzugt zu Thiosulfonsäure-S-estern und Sulfonsäuren disproportionieren.

Deshalb wurde es auch nach dem Stand der Technik für notwendig erachtet, Chlormethylsulfone durch Umsetzung der Metallsalze von Sulfinsäuren und Dichloracetaten zu erhalten, um offensichtlich solche Disproportinierungsreaktionen auszuschließen.

Ein weiterer Vorteil des erfindungsgemäßen Verfahrens besteht darin, daß bei der Umsetzung mit freier Dichloressigsäure im sauren pH-Bereich gearbeitet werden kann, wobei das durch Decarboxylierung des Zwischenproduktes enstehende Kohlendioxid aus dem Reaktionsgemisch gasförmig entweicht, während nach der Arbeitsweise des Standes der Technik dieses als Carbonat im Reaktionsprodukt verbleibt und von diesem erst zusätzlich abgetrennt werden müßte.

In einer besonderen Ausführungsform des Verfahrens gelingt es auch den zweiten Verfahrensschritt separat auszuführen. Dazu kann man z.B. die reinen Natriumsalze von zuvor isolierten Sulfobetainsulfinsäuren in Gegenwart von Jodid mit der moläquivalenten Menge Dichloressigsäure direkt zur Umsetzung bringen. Man kann aber auch Dichloracetate in analoger Weise mit isolierten Sulfobetainsulfinsäuren behandeln. In allen Fällen sind aber die so erhaltenen Reaktionsprodukte mit jenen identisch, welche nach dem zweistufigen Syntheseverfahren gewonnen werden. Dieser Syntheseweg bietet keinen prinzipiellen Verfahrensvorteil, da die dafür notwendigen Ausgangs-Sulfobetainsulfinate zuvor aus den Diallylammoniumsalzen hergestellt werden müssen.

Zusammenfassend besteht der Vorteil der Erfindung in einer einfachen Synthese, welche ohne die Isolierung von Zwischenprodukten die bisher unbekannten Chlormethylsulfon-sulfobetaine der Formel I aus Diallylammoniumsalzen direkt zugänglich macht. Als Ausgangsstoffe werden im technischen Maßstab verfügbare Zwischenprodukte genutzt, welche mit einfachen Mitteln in kurzen Reaktionszeiten zur Umsetzung gebracht werden können.

Die Erfindung soll durch die nachfolgenden Beispiele näher erläutert werden.

Beispiel 1

1,1-Dimethyl-3-chlormethylsulfonylmethyl-4-sulfomethyl-pyrrolidinium-betain
($R_1$ = $R_2$ = $CH_3$ in der allgemeinen Formel I)

a) In einem 1,5 1 Sulfierkolben, der mit einem Rührer, Thermometer, Rückflußkühler sowie einer Heizquelle ausgerüstet ist, werden 307,4 g (1 mol) 52,6 %ige wäßrige Dimethyldiallylammoniumchloridlösung, 129 g (1 mol) Dichloressigsäure sowie 539 g (2,02 mol) 39 %ige technische Natriumhydrogensulfitlösung vorgelegt und miteinander unter Rühren vermischt. Man erhält eine durch teilweise ungelöst gebliebene Dichloressigsäure leicht trübe Lösung von 21 °C und einen Start-pH-Wert von 1,7. Zur Initiierung der Umsetzung fügt man nun zunächst 4,56 g (2 Mol-%) Ammoniumperoxodisulfat und 1,5 min später - die Reaktionslösung hat sich inzwischen rot gefärbt und die Reaktionstemperatur ist auf 55 °C gestiegen - nochmals 2 Mol-% Ammoniumperoxodisulfat hinzu. Bereits nach einer weiteren Minute

wird das Temperaturmaximum von 71 °C erreicht. Man fügt zur Reaktionslösung 2,55 g (17 mmol) Natriumjodid hinzu und erwärmt für 3 Stunden zum Sieden auf 110 °C. Schon während des Hochheizens entfärbt sich die rote Reaktionslösung und ist bei Erreichen der Siedetemperatur fahlgelb. Die Umsetzung ist dann quantitativ beendet, wenn das Reaktionszwischenprodukt, 1,1-Dimethyl-3-sulfinatomethyl-4-sulfomethyl-pyrrolidinium-betain, durch bromatometrische Titration einer Probe der reagierenden Lösung nicht mehr nachweisbar ist . Man läßt abkühlen und zur vollständigen Kristallisation über Nacht stehen. Zur Aufarbeitung saugt man den Kristallbrei von der Mutterlauge scharf ab, wäscht die Kristalle mit wenig Wasser und zweimal mit Alkohol und erhält nach dem Trocknen an der Luft 200 g eines weißen, lockeren Kristallpulvers, welches zur Feinreinigung noch aus Wasser umkristallisiert werden kann. F:ab 270 °C unter Zersetzung. Durch Aufarbeitung der Mutterlauge kann noch eine weitere Fraktion des Zielproduktes erhalten werden. $^{13}C$-NMR-Spektrum ($H_2O$/HCl; externer Standard Tetramethylsilan = TMS; $\delta$ = 0,0 ppm):
Die Zahlenangaben an den Atomsymbolen entsprechen den chemischen Verschiebungen für die cis-Konfiguration (3,4-Position) in ppm.

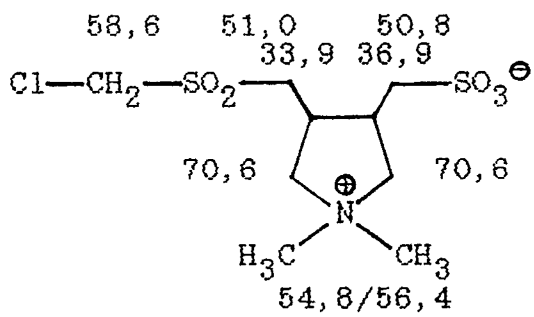

Die N-$CH_3$-Gruppen sind nicht äquivalent und wie bei den N-$CH_2$-Gruppen erfolgt noch zusätzlich Signalaufspaltung durch das $^{14}N$-Quadrupolmoment.
b) 307 g (1 mol) 1,1-Dimethyl-3-sulfinsäuremethyl-4-sulfomethyl-pyrrolidinium-betain-dihydrat (hergestellt aus Dimethyldiallylammoniumchlorid und Natriumhydrogensulfit;vgl.DD 225 128 A1 Beispiel 13), 121,2 g (1 mol) 33 %ige Natronlauge, 129 g (1 mol) Dichloressigsäure, 3 g (20 mmol) Natriumjodid und 200 g Wasser werden miteinander vermischt und 3 Stunden zum Sieden erwärmt. Aus der farblosen Reaktionslösung fallen schon nach kurzer Zeit Kristalle des Zielproduktes aus. Nach Abkühlung der Reaktionslösung können 302 g (94,5 % Ausbeute) an Chlormethylsulfon-sulfobetain isoliert werden. Das $^{13}C$-NMR-Spektrum des erhaltenen Produkts ist mit dem nach Beispiel 1a hergestellten völlig identisch.

Beispiel 2

3-Chlormethylsulfonylmethyl-4-sulfomethyl-pyrrolidinium-betain
($R_1$ = $R_2$ = H in der allgemeinen Formel I)

Man verfährt nach Beispiel 1a und setzt eine Mischung vom pH 2,3 aus 19,4 g (0,2 mol) Diallylamin, 107,8 g (0,404 mol) 39 %ige technische Natriumhydrogensulfitlösung und 25,8 g (0,2 mol) Dichloressigsäure mit zwei Portionen von je 0,92 g (2 Mol-%) Ammoniumperoxodisulfat um. Dabei steigt die Reaktionstemperatur von 27 auf 65 °C an. Man fügt 0,45 g (3 mmol) Natriumjodid hinzu und erwärmt für 3 Stunden zum Sieden und engt die Reaktionslösung zu einer viskosen, hellbraunen Masse ein. Nach Durcharbeiten mit Methanol sowie Trocknen an der Luft werden 43 g beigefarbene Kristalle erhalten. F. ab 200 °C unter Zersetzung.
$^{13}C$-NMR-Spektrum ($D_2O$, externer Standard TMS = 0,0 ppm)

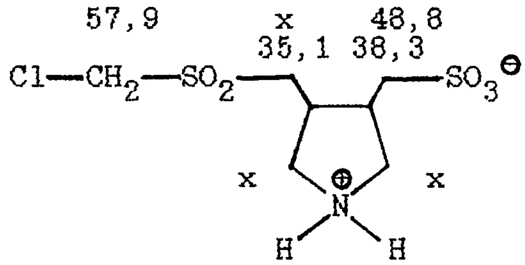

x: 49,3; 49,7; 50,0 ppm.

Beispiel 3

1-Dodecyl-3-chlormethylsulfonylmethyl-4-sulfomethyl-pyrrolidinium-betain ($R_1$ = H; $R_2$ = $C_{12}H_{25}$ in der allgemeinen Formel I)

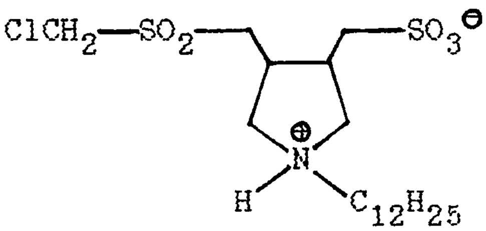

I

Man verfährt nach Beispiel 1a und setzt eine Mischung von pH 2,2 aus 26,55 g (0,1 mol) Diallyldodecylamin, 12,9 g (0,1 mol) Dichloressigsäure, 53,9 g ( 0,202 mol) 39 %ige technische Natriumhydrogensulfitlösung und 3 g 37 %ige Salzsäure mit zwei Portionen von je 0,46 g (2 Mol-%) Ammoniumperoxodisulfat um. Dabei steigt die Reaktionstemperatur von 28 °C auf 69 °C an; die Lösung wird viskos. Nach Zusatz von 0,3 g (2 mmol) Natriumjodid sowie 3stündigem Erwärmen zum Sieden färbt sich die anfänglich zitronengelbe Lösung bräunlich (Freisetzung von Jod; Lösung kann vollständig durch Zusatz weniger Tropfen Hydrogensulfitlösung entfärbt werden) und setzt sich vollständig zu einer viskosen, schäumenden Lösung des Zielproduktes um.

**Patentansprüche**

1. 3-Chlor-methylsulfonylmethyl-4-sulfomethyl-pyrrolidinium-betaine der allgemeinen Formel I,

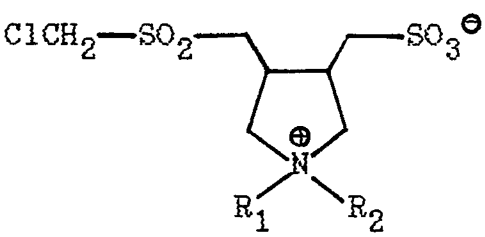

I

worin
$R_1$ und $R_2$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen.

2. Verbindung nach Anspruch 1, worin $R_1$ und $R_2$ $-CH_3$ bedeuten.

3. Verbindung nach Anspruch 1, worin $R_1$ und $R_2$ Wasserstoff bedeuten.

4. Verbindung nach Anspruch 1, worin $R_1$ Wasserstoff und $R_2$ $C_{12}H_{25}$ bedeuten.

5. Verfahren zur Herstellung von 3-Chlor-methylsulfonylmethyl-4-sulfomethyl-pyrrolidinium-betainen der allgemeinen Formel I nach Anspruch 1 bis 4, wobei Diallylammoniumsalze, vorzugsweise Diallylammoniumchloride, der allgemeinen Formel II,

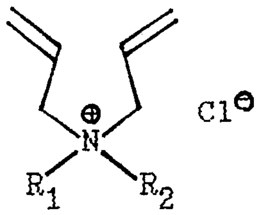

II

in der
$R_1$ und $R_2$ die obengenannten Bedeutungen haben,
mit molaren Mengen Dichloressigsäure und der zweifachmolaren Menge eines Metallhydrogensulfits, vorzugsweise Natriumhydrogensulfit,in Gegenwart einer katalytischen Menge eines Peroxodisulfats im ersten Verfahrensschritt miteinander umgesetzt und im zweiten Verfahrensschritt die erhaltene Reaktionslösung nach Zugabe einer katalytischen Jodidmenge solange zum Sieden erhitzt wird bis der Sulfinat-Gehalt der reagierenden Lösung auf Null abgesunken bzw. die Decarboxylierung beendet ist.

6. Verfahren nach Anspruch 5, gekennzeichnet dadurch, daß im ersten Verfahrensschritt 1 bis 5 Mol-% Peroxodisulfat, bezogen auf das eingesetzte Diallylammoniumsalz, als Katalysator zugesetzt werden.

7. Verfahren nach Anspruch 5 und 6, gekennzeichnet dadurch, daß das Peroxodisulfat in zwei Portionen von jeweils 2 Mol-%, bezogen auf das eingesetzte Diallylammoniumsalz, zugesetzt wird.

8. Verfahren nach Anspruch 5, gekennzeichnet dadurch, daß im zweiten Verfahrensschritt 14 bis 20 mmol Natrium-, Kalium- oder Ammoniumjodid/mol Diallylammoniumsalz als Katalysator zugesetzt werden.

Europäisches Patentamt

# EUROPÄISCHER RECHERCHENBERICHT

Nummer der Anmeldung

**EP 90 25 0323**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | GB-A-2 080 293 (AKADEMIE D. WISSENSCHAFTEN)<br>* Insgesamt *<br>- - - | 1-8 | C 07 D 207/08 |
| A | US-A-4 528 383 (SCHMITT)<br>* Insgesamt *<br>- - - | 1-8 | |
| D,A | EP-A-0 163 319 (AKADEMIE D. WISSENSCHAFTEN)<br>* Insgesamt *<br>- - - - - | 1-8 | |
| | | | RECHERCHIERTE SACHGEBIETE (Int. Cl.5) |
| | | | C 07 D 207/00 |

Der vorliegende Recherchenbericht wurde für alle Patentansprüche erstellt

| Recherchenort | Abschlußdatum der Recherche | Prüfer |
|---|---|---|
| Den Haag | 15 März 91 | KISSLER B.E. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument